## Europäisches Patentamt

## European Patent Office

## Office européen des brevets

(19)

(11) Publication number: **0 001 698**
**B1**

(12) **EUROPEAN PATENT SPECIFICATION**

(45) Date of publication of patent specification: **21.10.81**

(51) Int. Cl.³: **C 07 C 37/11, C 07 C 39/06**

(21) Application number: **78300480.7**

(22) Date of filing: **10.10.78**

(54) Isomerisation and production of alkylphenols.

(30) Priority: **13.10.77 US 841625**
**13.10.77 US 841626**

(43) Date of publication of application:
**02.05.79 Bulletin 79/9**

(45) Publication of the grant of the European patent:
**21.10.81 Bulletin 81/42**

(84) Designated Contracting States:
**BE CH DE FR GB NL**

(56) References cited:
**GB - A - 1 258 727**
**GB - A - 1 287 640**

**Chemical Abstracts Vol, 80, no. 9,
1974 abstract no. 47622w
Y. SUZUKI et al
page 330, column 1 to 2**

(73) Proprietor: **STAUFFER CHEMICAL COMPANY
Westport Connecticut 06880 (US)**

(72) Inventor: **Giolito, Silvio Louis
6-19 150th Place
Whitestone N.Y. 11357 (US)**
Inventor: **Mirviss, Stanley Burton
90 Surrey Road
Stamford Connecticut 06903 (US)**

(74) Representative: **Smith, Sydney et al,
Elkington and Fife High Holborn House 52/54
High Holborn
London WC1V 6SH (GB)**

Courier Press, Leamington Spa, England.

Isomerisation and production of alkylphenols

The present invention relates to the preparation of alkylphenols having reduced concentrations of 2,6-dialkylphenol isomer, and increased concentrations of meta-alkylphenol isomers by isomerisation of alkylphenols and mixtures wherein the alkylphenols may be prepared in a first stage of a two-stage process. Such alkylphenols are particularly useful in preparing low-colour phosphate esters.

The most common method for preparing alkylphenols comprises the alkylation of phenol with an olefin in the presence of a Friedel-Crafts catalyst. The product of this alkylation reaction generally comprises a mixture of several isomeric forms of the alkylphenol. These mixtures often contain little or no meta-isomer. For many purposes, however, the meta-isomers are more desirable than either the ortho- or para-isomers.

In addition, the mixtures often contain 2,6-dialkylphenols which are a cause of undesirable colour in phosphate esters made from the alkylphenols.

It is therefore desirable to be able to prepare alkylphenols having reduced concentrations of 2,6-dialkylphenols and increased concentration of meta-alkylphenol.

Isomerisation of alkylphenols is known from United States Patents 3,014,079 and 3,932,537 as well as from United Kingdom Patent 1,112,502.

In United States Patent No. 3,932,537 there is described an improvement in a method for producing para-alkylated phenols comprising reacting (A) a phenol and (B) an alkylating agent in the presence of (C) an alkylation catalyst, wherein one uses as catalyst an aryl sulfonic acid which is selected from the class of highly acidic aryl sulphonic acids having a K value of at least $3.8 \times 10^{-3}$ including certain specified acids including methane disulfonic acid and trifluoromethanesulfonic acid.

It is stated that these acid catalysts are also very effective in rearranging ortho- and meta-substituted phenols to parasubstituted phenols allowing use of normally discarded by-products as starting materials.

In Example (V) 564 gms of phenol containing 5 gms of trifluoromethanesulfonic acid catalyst is stirred at 90°C and butylated with isobutylene to a degree of 0.92. The batch is then heated to 120°C with stirring. Analysis of the crude product is said to give 84% weight paratetriarybutyl phenol.

We have now suprisingly found that the use of trifluoromethane sulfonic acid as the catalyst under certain circumstances promotes the isomerisation of 2,6-dialkylphenol to other isomeric forms and promotes the isomerisation of nonmeta-isomers to meta-isomers. This is contrary to the teaching of the prior patent just referred to. Other strong acids such as meta-benzene disulphonic acid are not effective in promoting this isomerisation. The process of the present invention depends upon the use of more severe conditions than those used in the prior art and the use of a particular catalyst.

According to the present invention there is provided a method for reducing the 2,6-dialkylphenol content of an alkylphenol mixture and increasing the meta-alkylphenol content thereof which comprises forming an isomerisation mixture of the alkylphenol mixture and a catalytically-effective amount of trifluoromethane sulphonic acid catalyst; and a maintaining the isomerisation mixture at a temperature ranging from 120°C, to the reflux temperature of the isomerisation mixture for a period of time sufficient to reduce the 2,6-dialkylphenol content and increase the meta-alkylphenol content and recovering a product of reduced 2,6-dialkylphenol content and increased meta-alkylphenol content.

The alkylphenol which is isomerised may be a pre-prepared commercial product or may be formed in an earlier step of the process by alkylating phenol in the presence of a relatively low concentration of trifluoromethane sulphonic acid as catalyst. The product so formed already has a reduced concentration of 2,6-dialkylphenol and an increased concentration of meta-alkylphenol.

In accordance with a preferred aspect of the invention there is provided a method of forming alkylphenol mixtures comprising the steps of forming a reactant mixture comprising phenol, an olefin, and a catalytically-effective amount of trifluoromethane sulphonic acid catalyst; and reacting the reactant mixture at a temperature ranging from 120°C. to the reflux temperature of the reactant mixture to form an alkylphenol reaction product; and maintaining the alkylphenol reaction product at a temperature ranging from 120°C. to the reflux temperature of the alkylphenol reaction product for a period of time sufficient to form an alkylphenol mixture having the desired concentration of 2,6-dialkylphenol and/or meta-alkylphenol.

In a preferred embodiment, isopropylphenol mixtures containing less than 2% by weight of 2,6-di-isopropylphenol and more than 2% by weight meta-isopropylphenol are prepared by forming a reactant mixture comprising phenol, propylene, and trifluoromethane sulphonic acid catalyst wherein the molar ratio of propylene/phenol ranges from 0.2:1 to 2:1 and the concentration of trifluoromethane sulphonic acid ranges from 0.01% to 5.0% by weight of reactant mixture; and reacting the reactant mixture at a temperature ranging from 120°C. to the reflux temperature of the reactant mixture to form an isopropylphenol reaction product; and maintaining the isopropylphenol reactant product at a temperature ranging from 120°C. to the reflux temperature of said isopropylphenol reaction product for a period of time sufficient to form an isopropylphenol mixture having less than 2% by weight of 2,6-diisopropylphenol and more than 2% by weight meta-isopropylphenol.

The olefins which are most advantageously reacted with phenol to form alkylphenol mixtures include, but are not limited to, those olefins, either branched or linear, having from 2 to 12 carbon atoms such as ethylene, propylene, butylene, amylene, tripropylene, tetrapropylene, decene, dodecene, diisobutylene and their isomers and mixtures thereof. Particularly preferred olefins are propylene and isobutylene of which propylene is most preferred.

The molar ratio of olefin/phenol used in preparing the alkylphenol is not a critical factor, and appropriate molar ratios for the desired alkylphenol mixture will be easily selected by those skilled in the art. In general, however, the molar ratio of olefin/phenol in the reaction mixture will range from 0.2:1 to 2:1 We have found that in preparing isopropylphenol mixtures which are to be subsequently phosphorylated to form phosphate esters for use as plasticizers, an olefin/phenol mole ratio ranging from 0.3:1 to 0.6:1 produces particularly good results. When reacted in accordance with the process of this invention, a reaction mixture containing olefin and phenol in this molar ratio generally produces a mixture of isopropylphenols and unreacted phenol which may then be phosphorylated to produce a phosphate ester.

The amount of trifluoromethane sulphonic acid catalyst in the reaction and isomerisation mixtures which is catalytically effective is a concentration of trifluoromethane sulphonic acid catalyst varying from 0.01% to 5% or more by weight of the initial mixture that is the reaction or isomerisation mixture. Although no particular concentration of trifluoromethane sulphonic acid has been found to be critical in the preparation of the alkylphenols it has been found that at concentrations below 0.01% by weight the reaction proceeds so slowly as to be impractical; while at concentrations above 1%, the benefits of increased reactivity are out-weighed by the increased cost of the trifluoromethane sulphonic acid. For most applications in accordance with the invention it has been found that a trifluoromethane sulphonic acid concentration ranging from 0.025 to 1.0% by weight of the initial mixture produces a reasonable reaction rate at a reasonable cost.

The trifluoromethane sulphonic acid may be added to the reaction or isomerisation mixtures by any of those techniques known in the art for contacting chemical reactants with catalysts. Since trifluoromethane sulphonic acid is readily soluble in phenol and many alkylphenols, a preferred technique is to add the trifluoromethane sulphonic acid directly to the reaction mixture in liquid form. The trifluoromethane sulphonic acid may be added "neat" or as a solution in phenol, in the alkylphenol being isomerized or in other appropriate solvent. Since the total amount of trifluoromethane sulphonic acid required is generally quite small, it will be found most convenient to add it to the reaction mixture as a dilute solution.

When used in the form of a liquid which is miscible with the other components of the reaction mixture, the trifluoromethane sulphonic acid may be easily added or continuously metered to the reaction mixture. When used in this form, the trifluoromethane sulphonic acid may also be readily removed from the product upon completion of the reaction. Thus, for example, upon completion of the reaction, the trifluoromethane sulphonic acid may be left unneutralized or neutralized with a convenient base. When the reaction mass is subsequently purified by distillation, the neutralized trifluoromethane sulphonic acid will remain in the usual distillation bottoms, which are discarded after each fractionation. Moreover, since the crude reaction mass does not have to be washed to remove spent catalyst, there are no large amounts of waste water to be disposed of.

The trifluoromethane sulphonic acid may also be added to the reaction or isomerisation mixture in the form of a heterogeneous catalyst such as that formed by impregnating a clay or other particulate substrate with the trifluoromethane sulphonic acid or adsorbing the trifluoromethane sulphonic acid onto a clay or other particulate substrate. The advantages offered by this type of catalyst such as, for example, the ability to recover and recycle the catalyst, often outweigh the inconveniences associated with the preparation and use of heterogeneous catalysts.

Where the isomerisation mixture is formed in a first stage of the process as is preferred the formation of the alkylphenol in accordance with the practice of this invention is thought to take place in two distinct phases. In the first phase, chemical addition of the olefin to the phenol occurs resulting in the formation of a mixture of alkylphenols containing some 2,6-dialkylphenol and meta-alkylphenol isomers. In the second phase, this mixture of alkylphenols is isomerised to increase the content of meta-alkylphenol.

The time required to complete the first phase is dependent, in part, upon the rate at which the olefin may be absorbed into and reacted with the phenol. Thus, for example, when the olefin used is propylene, the time required to complete the reaction will be controlled, to a large extent, by the rate at which the gaseous propylene can be absorbed into the phenol liquid as well as the catalyst concentration and reaction temperature. This first stage may be referred to as the alkylation stage.

After all of the olefin has been added to the reaction mixture, the second phase of a reaction, referred to hereinafter as the "isomerisation phase", takes place. During the isomerisation phase of the reaction, the reaction mixture is maintained at an elevated temperature for a period of time sufficient to form the desired alkylphenol mixture.

During the isomerisation phase, the 2,6-dialkylphenols which were formed during the first phase of this reaction are isomerised to other isomeric forms of dialkylphenols and non-meta isomers are isomerised to meta-isomers. As will be understood by those skilled in the art, the time required to complete this second phase will depend on a number of factors. Thus, for example, the time required to complete the isomerisation phase of the reaction can be affected by catalyst (trifluoromethane sulphonic acid) concentration, reaction temperature, the particular alkyl groups present, and other such factors. Satisfactory results may generally be achieved in time periods ranging from 1 to 36 hours although, where it is desired to approach equilibrium conditions, time periods of up to 50 hours or more may be required.

The reflux temperature of the reaction or isomerisation mixture may, of course, vary in accordance with the pressure at which the reaction or isomerisation is being conducted. The pressure, in turn, may vary from vacuo to several atmospheres depending on the needs of the individual practitioner. Thus, the reaction time may be reduced to some extent by conducting the reaction at higher reflux temperatures possible at elevated pressures.

When practicing the method of this invention in conjunction with isopropylphenol or tertiary-butylphenol mixtures, the reflux temperatures of the reaction mixtures, reaction products, and isomerisation mixtures range 185°C. to 190°C. at atmospheric pressure.

The alkylphenol mixtures in the process of this invention contain a number of isomeric alkylphenol components as well as unreacted phenols. Thus, as will be shown in the examples which follow, these product mixtures may contain phenol, ortho-alkylphenol, meta-alkylphenol, para-alkylphenol, 2,6-dialkylphenol, 2,4-dialkylphenol, 3,5-dialkylphenol and 2,5-dialkylphenol.

In a typical preparation of an alkylphenol mixture in accordance with the preferred practice of this invention, an appropriate quantity of phenol is added to a reaction vessel equipped with means for addition of the olefin, a stirrer, and a reflux condenser. A predetermined amount of trifluoromethane sulphonic acid is then added to the phenol and the mixture heated to a temperature of approximately 150°C. with stirring until all of the phenol has been melted. When all of the phenol has been melted, the olefin is then added at a rate which is dependant upon the rate of absorption of the particular olefin into the reaction mixture. Thus, for example, when the olefin is propylene, the addition rate may be determined by multiplying the absorption rate of propylene ($50—15 \times 10^{-4}$ g/g/min.) by the amount of phenol in the reactor. Once all the olefin has been added to the reactor, the temperature of the reaction mixture may be brought up to reflux and maintained at reflux temperature for a time sufficient to form an alkylphenol mixture having the desired low level of 2,6-dialkylphenol isomer and/or the desired content of meta-alkylphenyl. In the case of isopropylphenol, a product mixture having less than 2% of the 2,6-diisopropylphenol isomer can generally be prepared in a period of 8 to 12 hours after reflux has been initiated.

However, the isomerisation phase may be carried out separately from the alkylation phase and this is within the broad scope of the invention. Thus it may be desirable to isomerise an alkylphenol mixture, a pure 2,6-dialkylphenol or a pure non-meta-alkylphenol which may have been prepared by a method different from that described above for the alkylation stage or indeed may have been prepared by the first stage of the process and isolated before subsequent processing.

As indicated in the preferred process according to the invention the alkylphenol is produced in an alkylation phase and the product is then isomerised in an isomerisation phase.

The process of the invention may, of course, be operated as a continuous as well as a batch process.

The invention will be further illustrated by the following examples.

Example 1

*Preparation of isopropylphenol*

A 470-gram charge of crystalline synthetic phenol was added to a 1-litre, 3 neck flask equipped with a stirrer and reflux condenser. Trifluoromethane sulphonic acid in the amount of 0.235 gram was added to the reactor which was then heated to 150°C. with stirring. The reactor was held at 150°C. while 105 grams of propylene were added at the rate of 4.8 grams per minute. Previous experimentation had indicated that the absorption rate of propylene into phenol at this temperature was on the order of $102 \times 10^{-4}$ g/g/min.

After all of the propylene was added to the reaction mixture, the temperature of the charge was raised to the atmospheric reflux temperature (approximately 190°C.) and held there for a total of 12 hours in order to isomerise the reaction mixture. The isomerisation progress was monitored by analyzing samples of the reaction mixture just prior to raising the temperature to 190°C. (0 hour) and after 8 hours and 12 hours, respectively, at that temperature. The results of these analyses are shown below.

4

TABLE I

| *GC Composition | 0 Hour | 8 Hours | 12 Hours |
|---|---|---|---|
| Phenol | 45.2 | 40.7 | 41.7 |
| Ortho-isopropylphenol | 26.3 | 25.6 | 22.2 |
| Meta- + para-isopropylphenol | 10.9 | 21.0 | 22.3 |
| 2,6-diisopropylphenol | 6.4 | 2.5 | 1.7 |
| 2,4-diisopropylphenol | 7.0 | 8.5 | 8.0 |
| Unknown | 1.3 | —— | —— |
| 3,5 + 2,5 diisopropylphenol | 0.3 | 1.3 | 1.9 |
| Unknown | 2.1 | 0.4 | 0.5 |

*Compositions determined by gas chromatography. Amounts are in percentages by weight.

This test shows that the 2,6-diisopropylphenol content was reduced to less than 2% by weight over the 12 hour period.

Example 2

*Preparation of isopropylphenyl diphenyl phosphate*

Phosphorus oxychloride in the amount of 212 grams and anhydrous magnesium chloride catalyst in the amount of 1 gram were added to 485 grams of the isopropylphenol mixture prepared in Example 1 under the heading "12 hours". The charge was gradually heated with stirring from 20°C. to 180°C. over a 10-hour period. The evolution of HCl gas was noted during heating. The reaction mixture was held at 180°C. until the evolution of HCl gas ceased. At this point, infra-red analysis indicated the reaction to be complete. The crude ester weighed 550 grams (theoretical 546 grams.)

The 550 grams of crude ester was distilled through a 15.25 × 2.54 cms diameter Vigreaux column to produce 477 grams of distillate having a boiling point between 178°C. and 198°C. at 1.0 mm. Hg., and 35 grams of residue. The distillate was washed twice with 500 ml of 2% caustic solution for one hour at 65°C. The ester was then washed with a volume of water equal to that of the ester at 65°C. for 30 minutes.

The washed ester was vacuum dried at 70°C. and 1.0 mm. Hg. for one hour and then filtered through a thin layer of dry Celite (Celite is a Registered Trade Mark) filter-aid to produce 463 grams of product having the following properties:

| | |
|---|---|
| Refractive Index ($N_D^{25}$) | 1.5485 |
| Density ($d_{25}$) | 1.13 |
| Acid Number | 0.03 |
| APHA Colour | 50 |

The phosphate ester product was found to have good thermal stability (i.e., lack of colour formation in a one-hour milling test in PVC in the presence of air. This product also demonstrated good storage stability upon ageing in the dark for several weeks in the presence of air.

Example 3

*Isomerisation of commercial isopropylphenyl*

A sample of isopropylphenol prepared by a commercial process was found to have the following initial analysis:

TABLE II

| Composition | Weight Percent |
|---|---|
| Phenol | 53.8 |
| Ortho-isopropylphenol | 25.5 |
| Meta- + para-isopropylphenol | 7.3 |
| 2,6-diisopropylphenol | 5.3 |
| 2,4-diisopropylphenol | 5.2 |
| 2,5 + 3,5 diisopropylphenol | 0.7 |
| Uknown | 1.4 |
| Triisopropylphenol | 0.2 |

0.4 gram of trifluoromethane sulphonic acid was added to 400 grams of the above-described isopropylphenol mixture, and the charge was heated for 8 hours at atmospheric reflux temperature. Samples taken at 4 and 8 hours were analyzed as in Example 1; the results of these analyses are shown in Table III.

TABLE III

| Composition | Weight % After 4 Hrs. | Weight % After 8 Hrs. |
|---|---|---|
| Phenol | 48.3 | 48.8 |
| Ortho-isopropylphenol | 20.3 | 17.5 |
| Meta- + para-isopropylphenol | 23.1 | 26.1 |
| 2,6-diisopropylphenol | 1.2 | 0.9 |
| 2,4-diisopropylphenol | 6.5 | 5.6 |

Example 4

*Preparation of isopropylphenyl diphenyl phosphate*

Phosphorus oxychloride in the amount of 169 grams and anhydrous magnesium chloride catalyst in the amount of 1 gram were added to 395 grams of the isopropylphenol mixture prepared in Example 3. This mixture was reacted and then distilled and refined in accordance with the procedure described in Example 2.

The final product weighed 368 grams and had the following properties.

| | |
|---|---|
| Refractive Index ($N_D^{25}$) | 1.5500 |
| Density ($d_{25}$) | 1.140 |
| Acid Number | 0.01 |
| APHA Colour | 50 |

As in the case of Example 2, the ester product demonstrated good thermal stability as well as good storage stability.

Example 5

An isopropylphenol mixture was isomerised in the presence of 0.05% by weight of trifluoromethane sulphonic acid for a period of 18 hours at 185°C. Samples of the isopropylphenol mixture were analyzed both before and after the isomerisation. The results of this analysis are tabulated below in table IV.

6

## TABLE IV

| Component | Before (% by Wt.) | After (% by Wt.) |
|---|---|---|
| Phenol | 47.2 | 45.5 |
| Ortho-isopropylphenol | 24.9 | 21.4 |
| Meta-isopropylphenol | 0.9 | 2.8 |
| Para-isopropylphenol | 12.5 | 18.8 |
| 2,6-diisopropylphenol | 5.3 | 1.7 |
| 2,4-diisopropylphenol | 6.3 | 6.8 |
| 2,5- + 3,5-diisopropylphenol | 1.3 | 2.2 |
| Triisopropylphenol | 1.6 | 0.8 |

This shows that the content of 2,6-diisopropylphenol was reduced to less than 2%, and the content of meta-alkylphenol was increased from the initial 0.9% to 2.8%.

Example 6

An isopropylphenol mixture was divided into three parts. The separate parts were then isomerised at 190°C. in the presence of trifluoromethane sulphonic acid at concentrations of 0.10% by weight, 0.30% by weight, and 0.50% by weight respectively. Each sample was analyzed after 4 hours and 8 hours of isomerisation. The results are shown below in Table V.

## TABLE V

| Composition | 0.10% by Weight TFMSA* | | |
|---|---|---|---|
| | Initial | 4 Hours | 8 Hours |
| Phenol | 47.6 | 42.9 | 45.2 |
| Ortho-isopropylphenol | 25.9 | 26.3 | 22.9 |
| Meta- + para-isopropylphenol | 9.8 | 17.9 | 20.7 |
| 2,6-diisopropylphenol | 6.2 | 3.4 | 1.8 |
| 2,4-diisopropylphenol | 6.1 | 6.8 | 6.8 |
| 2,5- + 3,5-diisopropylphenol | 1.2 | 1.5 | 1.5 |

*Trifluoromethane sulphonic acid.

## TABLE V — Continued

| Composition | 0.30% by Wt. TFMSA | | 0.50% by Wt. TFSMA | |
|---|---|---|---|---|
| | 4 Hours | 8 Hours | 4 Hours | 8 Hours |
| Phenol | 42.9 | 42.0 | 42.0 | 41.7 |
| Ortho-isopropylphenol | 17.9 | 14.5 | 16.4 | 13.8 |
| Meta-+ para-isopropylphenol | 28.4 | 32.9 | 30.8 | 34.3 |
| 2,6-diisopropylphenol | 0.8 | 0.6 | 0.7 | 0.5 |
| 2,4-diisopropylphenol | 6.8 | 5.6 | 6.3 | 5.4 |
| 2,5- + 3,5-diisopropylphenol | 2.2 | 2.8 | 2.3 | 3.0 |

### Example 7

A mixture of tertiary-butylphenol isomers (500 grams) was added to a 250 ml. round-bottom flask together with 1.0 gram (0.5 weight percent) trifluoromethane sulphonic acid. The charge was heated to reflux temperature (185°C.) while stirring and maintained at this temperature for 18 hours.

Samples of the original tertiary-butylphenol mixture and the final product after 18 hours at reflux were each analyzed by gas chromatography with the following results:

| Component | Original Mixture (Wt. Percent) | Final Product (Wt. Percent) |
|---|---|---|
| Phenol | 44.1 | 50.9 |
| Ortho-tertiary-butylphenol | 7.7 | 0.4 |
| Meta-tertiary-butylphenol | —— | 35.0 |
| Para-tertiary-butylphenol | 44.9 | 9.3 |
| 2,6-ditertiary-butylphenol | 0.1 | 0.2 |
| 2,4-ditertiary-butylphenol | 3.1 | 2.7 |
| 2,5- + 3,5 - tertiary- butylphenol | —— | 0.1 |
| Tritertiary-butylphenol | —— | 0.4 |
| Others | 0.1 | 1.0 |

This shows that the meta-isomer content was increased from essentially 0 to 35% over 18 hours.

### Example 8

A mixture of isopropylphenol isomers (100 grams) was added to a 250-ml. round bottom flask together with 2.0 grams (2% by weight) trifluoromethane sulphonic acid. This charge was heated to reflux temperature (185—190°C.) and maintained at this temperature for 18 hours.

Samples of the original isopropylphenol mixture and the final product after 18 hours at reflux were analyzed by gas chromatography with the following results:

| Component | Original Mixture (Wt. Percent) | Final Product (Wt. Percent) |
|---|---|---|
| Phenol | 47.1 | 37.8 |
| Unknown | —— | 0.6 |
| Ortho-isopropylphenol | 28.6 | 7.4 |
| Meta-isopropylphenol | 0.0 | 16.4 |
| Para-isopropylphenol | 7.2 | 15.1 |
| Unknown | —— | 0.3 |
| 2,6-diisopropylphenol | 6.9 | 0.3 |
| 2,4-diisopropylphenol | 6.3 | 3.6 |
| 2,5- + 3,5-diisopropylphenol | 1.2 | 4.4 |
| Others | 2.7 | 14.1 |

### Example 9

Pure para-tertiary-butylphenol in the amount of 100 grams was added to a 250-ml. round-bottom flask together with 1.0 gram (1% by weight) trifluoromethane sulphonic acid. The charge was then heated while stirring to reflux temperature (185°C—190°C) and maintained at this temperature for 18 hours. The resulting product was analyzed by gas chromatography with the following results:

| Component | % by Weight in Product |
|---|---|
| Phenol | 31.5 |
| Unknown | 2.3 |
| Ortho-tertiary-butylphenol | 0.7 |
| Meta-tertiary-butylphenol | 24.8 |
| Para-tertiary-butylphenol | 11.4 |
| Unknown | 4.3 |
| 2,6-ditertiary-butylphenol | 0.5 |
| Unknown | 2.0 |
| 2,4-ditertiary-butylphenol | 0.8 |
| 2,5- + 3,5-ditertiary-butylphenol | 6.8 |
| 2,4,6-tritertiary butylphenol | 2.1 |
| Others | 12.8 |

While these illustrative examples demonstrate the practice of this invention in conjunction with a batch-type process, it will be understood that the method of the invention may also be practiced in a continuous process and may be applied to single isomeric components as well as to mixtures.

As used herein, the term "alkylphenol" when not designated as a specific isomer, such as ortho- or para-, is defined as meaning either a single isomer or an isomeric mixture such as those which result from the Friedel-Crafts alkylation of phenol. As mentioned earlier, such mixtures may contain unreacted phenols, mono-isomer such as meta-, para- and ortho- as well as diisomers such as 2,6-; 2,4-; etc

It can be seen from the preceding examples that the method of this invention enables the effective and efficient preparation of alkylphenols having low concentrations of 2,6-dialkylphenol isomers and increased meta-alkylphenol content.

**Claims**

1. A process for the isomerisation of an alkylphenol mixture, a pure 2,6-dialkylphenol or a pure non-meta alkylphenol to produce or increase the content of the meta-isomer characterised in that the alkylphenol mixture, a pure 2,6-dialkylphenol or a pure non-meta alkylphenol is heated with trifluoromethane sulphonic acid in an amount of at least 0.01% by weight based on the initial mixture at a temperature within the range of 120°C to the reflux temperature of the alkylphenol component for a time sufficient to produce or increase the content of the meta-isomer.

2. A process as claimed in claim 1 characterised in that the concentration of the trifluoromethane sulphonic acid is from 0.01 to 5.0% by weight based on the weight of the initial mixture.

3. A process as claimed in either claim 1 or claim 2 characterised in that the alkylphenol mixture contains a 2,6-dialkylphenol and further characterised in that the mixture is an isopropylphenol or tertiarybutyl phenol mixture, and the process is carried out for a period sufficient to reduce the proportion of 2,6-dialkylphenol therein.

4. A process as claimed in any one of claims 1 to 3 characterised in that the alkylphenol mixture is prepared as a first step in the process by forming a reactant mixture comprising phenol, an olefin, and the trifluoromethane sulphonic acid catalyst and reacting the reaction mixture at a temperature within the range of 120°C to the reflux temperature of the reactant mixture to form the alkylphenol mixture.

5. A process as claimed in claim 4 characterised in that the alkylphenol mixture formed in the first step is subjected to the process of any of claims 1 to 2 without intermediate isolation.

6. A process as claimed in claim 5 characterised in that it consists of the following steps:

(a) forming a reactant mixture comprising phenol, propylene, and the trifluoromethane sulphonic acid catalyst wherein the molar ratio of propylene/phenol ranges from 0.2:1 to 2:1 and the concentra-

9

tion of the catalyst ranges from 0.01 to 5.0% by weight of the reactant mixture and reacting said reaction mixture at a temperature ranging from 120°C to the reflux temperature of said reactant mixture to form an isopropylphenol reaction product; and

(b) maintaining said isopropylphenol reaction product at a temperature ranging from 120°C to the reflux temperature of said isopropylphenol reaction product for a period of time sufficient to form an isopropylphenol mixture having less than 2% by weight of 2,6-diisopropylphenol isomer.

7. A process as claimed in claim 6 characterised in that the amount of trifluoromethane sulphonic acid is within the range of 0.025 to 1.0% by weight based on the weight of the reaction mixture.

8. A process as claimed in claim 7 characterised in that it is carried out at atmospheric pressure.

9. A process as claimed in claim 8 characterised in that the olefin and phenol are used in a molar ratio ranging from 0.2:1 to 2:1.

10. A process as claimed in claim 9 characterised in that the olefin is ethylene, propylene, butylene, amylene, tripropylene, tetrapropylene, decene, dodecene, or diisobutylene or isomers therof or mixtures thereof.

11. A process as claimed in claim 10 characterised in that the olefin is propylene and the temperature range for the production of the alkylphenol product is 120°C. to 190°C., and the temperature range of the isomerisation of the product according to the process of claim 1 is 120°C. to 200°C.

12. A process for the preparation of an isopropylphenol mixture containing less than 2% by weight of 2,6-diisopropylphenol and more than 2% by weight of meta-isopropylphenol comprising the steps of

(a) forming a reactant mixture comprising phenol, propylene, and trifluoromethane sulphonic acid catalyst wherein the molar ratio of propylene/phenol is within the range of 0.2:1 to 2:1 and the concentration of trifluoromethane sulphonic acid is within the range of 0.01 to 5.0% by weight of reactant mixture; and reacting said reactant mixture at a temperature within the range of 120°C to the reflux temperature of said reaction mixture to form an isopropylphenol reaction product; and

(b) maintaining said isopropylphenol reaction product at a temperature within the range of 120°C. to the reflux temperature of said reaction product for a time sufficient to form an isopropylphenol mixture having less than 2% by weight of 2,6-diisopropylphenol and more than 2% by weight of meta-isopropylphenol.

**Revendications**

1. Procédé d'isomérisation d'un mélange d'alkylphenols, de 2,6-dialkylphenols purs ou de non-meta alkylphenol pur en vue d'obtenir l'isomère meta pur ou une concentration augmentée, caractérisé en ce que l'on chauffe le mélange d'alkylphenols, le 2,6-dialkylphenol pur ou l'alkylphenol non-meta pur en présence d'acide trifluorométhane sulfonique à une concentration au moins égale à 0,01% en poids exprimée par rapport au mélange réactionnel initial, à une température comprise entre 120°C et la température de reflux de l'alkylphénol employé, pendant une durée suffisante pour obtenir ou augmenter la teneur de l'isomèreméta.

2. Procédé selon la revendication 1, caractérise en ce que la concentration en acide trifluoromethane sulfonique est comprise entre 0,01 et 5,0% en poids exprimé par rapport au mélange réactionnel initial.

3. Procédé selon la revendication 1 ou 2, caractérisé en ce que le mèlange d'alkylphenols renferme un 2,6-dialkylphenol et plus particulièrement en ce que le mélange est un mélange d'isopropylphenols ou de t-butylphenols et que l'on opère pendant une durée suffisante pour abaisser sa teneur en 2,6-dialkylphenol.

4. Procédé selon une quelconque des revendications 1 à 3, caractérisé en ce que l'on prépare le mélange d'alkylphenols, dans une première étape du procédé, en élaborant un mélange réactionnel constitué de phenol, d'une oléfine et d'acide trifluorométhane sulfonique en tant que catalyseur et en faisant réagir ce mélange à une température comprise entre 120°C et la température de reflux du mélange réactionnel jusqu'à l'obtention du mélange d'alkylphenols.

5. Procédé selon la revendication 4, caractérisé en ce que l'on soumet le mélange d'alkylphenol de la première étape à un procédé selon la revendication 1 ou 2 sans isolement intermédiaire.

6. Procédé selon la revendication 5, caractérisé en ce qu'il comporte les étapes suivantes:

(a) on forme un mèlange réactionnel renfermant du phenol, du propéne et de l'acide trifluoromethane sulfonique, où le rapport molaire du propène au phenol est compris entre 0,2:1 et 2:1 et la concentration du catalyseur est comprise entre 0,01 et 5,0% due poids du mélange réactionnel et on fait réagir ledit mélange à une température comprise entre 120°C et sa température de reflux afin d'obtenir, en tant que produit réactionnel, un mélange d'isopropylphenol;

(b) on maintient ledit produit de réaction de l'isopropylphénol à une température comprise entre 120°C et la température de reflux de l'isopropylphénol produit par la réaction jusqu'à ce que l'on obtienne un mèlange d'isopropylphénol renfermant moins de 2% en poids de 2,6-diisopropylphenol isomère.

7. Procédé selon la revendication 6, caractérisé en ce que la concentration en acide trifluorométhane sulfonique est comprise entre 0,025 et 1,0% du poids du mélange réactionnel.

8. Procédé selon la revendication 7, caractérisé en ce que l'on met en oeuvre le procédé à la pression atmosphérique.

9. Procédé selon la revendication 8, caractérisé en ce que l'on utilise un rapport molaire oléfine/phénol compris entre 0,2:1 et 2:1.

10. Procédé selon la revendication 9, caractérisé en ce que l'oléfine est l'éthylène, le propylène, le butène, l'amylène, le tripropylène, le tétrapropylène, le decène, le dodecène ou le diisobutylène, leurs isomères ou leurs combinaisons.

11. Procédé selon la revendication 10, caractérisé en ce que l'oléfine est le propène et la température de préparation de l'alkylphénol produit est comprise entre 120 et 190°C et la température d'isomerisation du produit selon la revendication 1 est comprise entre 120 et 200°C.

12. Procédé de préparation d'un mélange d'isopropylphénols renfermant moins de 2% en poids de 2,6-diisopropylphénol et plus de 2% en poids de méta-isopropylphénols, caractérisé en ce qu'il comporte les étapes suivantes:

(a) on forme un mélange réactionnel renfermant du phénol, du propène et de l'acide trifluorométhane sulfonique, où le rapport molaire du propène au phénol est compris entre 0,2:1 et 2:1 et la concentration en acide trifluorométhane sulfonique est comprise entre 0,01 et 5,0% du poids du mélange réactionnel puis, on porte le mélange à une température comprise entre 120°C et sa température de reflux afin d'obtenir un isopropylphénol comme produit de réaction; et

(b) on maintient ledit produit à une température comprise entre 120°C et la température de reflux dudit produit de la réaction pendant une durée de temps suffisante pour que se forme un mélange d'isopropylphénol renfermant moins de 2% en poids de 2,6 diisopropylphénol et plus de 2% en poids de méta-isopropylphénol.

## Patentansprüche

1. Verfahren zur Isomerisierung eines Alkylphenolgemisches, eines reinen 2,6-Dialkylphenols oder eines reinen Alkylphenols, das kein Metaalkylphenol ist, zur Herstellung oder Vergrößerung des Anteils des Metaisomers, dadurch gekennzeichnet, daß das Alkylphenolgemisch, ein reines 2,6-Dialkylphenol oder ein reines Alkylphenol, das kein Metaalkylphenol ist, mit Trifluormethansulfonsäure in einer Menge von mindestens 0,01 Gew.-%, bezogen auf das Ausgangsgemisch, bei einer Temperatur im Bereich von 120°C bis zur Rückflußtemperatur der Alkylphenolkomponente so lange erhitzt wird, wie dies zur Herstellung oder zur Vergrößerung des Anteils des Metaisomers erforderlich ist.

2. Verahren nach Anspruch 1, dadurch gekennzeichnet, daß die Konzentration der Trifluormethansulfonsäure 0,01 bis 5,0 Gew.-%, bezogen auf das Gewicht des Ausgangsgemisches, beträgt.

3. Verfahren nach einem der Ansprüche 1 oder 2, dadurch gekennzeichnet, daß das Alkylphenolgemisch ein 2,6-Dialkylphenol enthält und daß das Gemisch ein Isopropylphenolgemisch oder tert-Butylphenolgemisch ist, und daß das Verfahren ausreichend lange durchgeführt wird, um den im Gemisch enthaltenen Anteil des 2,6-Dialkylphenols zu verringern.

4. Verfahren nach einem der Ansprüche 1 bis 3, dadurch gekennzeichnet, daß das alkylphenolgemisch in einer ersten Stufe des Verfahrens hergestellt wird, in dem ein Ausgangskomponentengemisch hergestellt wird, das Phenol, ein Olefin und den Trifluormethansulfonsäurekatalysator enthält, und bei dem dieses Reaktionsgemisch bei einer Temperatur im Bereich von 120°C bis zur Rückflußtemperatur des Ausgangskomponentengemisches unter Bildung des Alkylphenolgemisches zur Umsetzung gebracht wird.

5. Verfahren nach Anspruch 4, dadurch gekennzeichnet, daß das Alkylphenolgemisch, das in der ersten Verfahrensstufe gebildet wird, dem Verfahren nach einem der Ansprüche 1 oder 2 unterworfen wird, ohne dazwischen isoliert zu werden.

6. Verfahren nach Anspruch 5, gekennzeichnet durch die folgenden Stufen:

(a) Herstellen eines Reaktantengemisches, das Phenol, Propylen und den Trifluormethansulfonsäurekatalysator enthält und in dem das Molverhältnis Propylen/Phenol im Bereich von 0,2 zu 1 bis 2 zu 1 liegt und die Konzentration des Katalysators im Bereich von 0,01 bis 5,0 Gew.-%, bezogen auf das Reaktantengemisch, liegt, und Umsetzen dieses Reaktionsgemisches bei einer Temperatur im Bereich von 120°C bis zur Rückflußtemperatur des Reaktantengemisches unter Bildung eines Isopropylphenolreaktionsproduktes; und

(b) Halten des so hergestellten Isopropylphenolreaktionsproduktes bei einer Temperatur im Bereich von 120°C bis zur Rückflußtemperatur des Isopropylphenolreaktionsproduktes für eine Zeit, die zur Bildung eines Isopropylphenolgemisches mit einem Gehalt von weniger als 2 Gew.-% 2,6-Diisopropylphenolisomer ausreicht.

7. Verfahren nach Anspruch 6, dadurch gekennzeichnet, daß der Anteil der Trifluormethansulfonsäure im Bereich von 0,025 bis 1,0 Gew.-%, bezogen auf das Gewicht des Reaktionsgemisches, liegt.

8. Verfahren nach Anspruch 7, dadurch gekennzeichnet, daß das Verfahren unter Atmosphärendruck durchgeführt wird.

9. Verfahren nach Anspruch 8, dadurch gekennzeichnet, daß das Olefin und das Phenol in einem Molverhältnis eingesetzt werden, das im Bereich von 0,2 zu 1 bis 2 zu 1 liegt.

10. Verfahren nach Anspruch 9, dadurch gekennzeichnet, daß das Olefin Ethylen, Propylen, Butylen, Amylen, Tripropylen, Tetrapropylen, Decen, Dodecen, Diisobutylen oder deren Isomer oder Gemische dieser Olefine ist.

11. Verfahren nach Anspruch 10, dadurch gekennzeichnet. daß das Olefin Propylen ist und die Temperatur zur Herstellung des Alkylphenolproduktes im Bereich von 120°C bis 190°C liegt, und daß die Temperatur, bei der die Isomerisierung dieses Produktes nach dem Verfahren nach Anspruch 1 durchgeführt wird, im Bereich von 120°C bis 200°C liegt.

12. Verfahren zur Herstellung eines Isopropylphenolgemisches, das weniger als 2 Gew.-% 2,6-Diisopropylphenol und mehr als 2 Gew.-% Metaisopropylphenol enthält, gekennzeichnet durch die folgenden Verfahrensstufen:

(a) Herstellen eines Reaktantengemisches, das Phenol, Propylen und Trifluoromethansulfonsäure als Katalysator enthält und in dem das Molverhältnis von Propylen zu Phenol im Bereich von 0,2 zu 1 bis 2 zu 1 liegt und in dem die Konzentration der Trifluormethansulfonsäure im Bereich von 0,01 bis 5,0 Gew.-%, bezogen auf das Reaktantengemisch, liegt, und Umsetzen dieses Reaktantengemisches bei einer Temperatur im Bereich von 120°C bis zur Rückflußtemperatur des Reaktionsgemisches unter Bildung eines Isopropylphenolreaktionsproduktes; und

(b) Halten des Isopropylphenolreaktionsproduktes bei einer Temperatur im Bereich von 120°C bis zur Rückflußtemperatur des Reaktionsproduktes für eine Zeit, die so ausreichend bemessen ist, daß ein Isopropylphenolgemisch gebildet wird, das weniger als 2 Gew.-% 2,6-Diisopropylphenol und mehr als 2 Gew.-% Metaisopropylphenol enthält.